# EUROPÄISCHE PATENTSCHRIFT

(11) **EP 3 827 882 B1**
(45) Veröffentlichungstag und Bekanntmachung des Hinweises auf die Patenterteilung: **08.04.2026**
(21) Anmeldenummer: 20210005.3
(22) Anmeldetag: 26.11.2020
(51) Int. Cl.: A61N 5/10, A61B 6/00

(54) **MULTILAMELLENKOLLIMATOR**
MULTILAMELLAR COLLIMATOR
COLLIMATEUR À LAMELLES MULTIPLES

(30) Priorität: 28.11.2019 DE 102019132299
(43) Veröffentlichungstag der Anmeldung: 02.06.2021
(73) Patentinhaber: gKteso MedSolutions GmbH, 90592 Schwarzenbruck (DE)
(72) Erfinder: GIEB, Klaus, 91058 Erlangen (DE); UELTZHÖFFER, Stefan, 92348 Berg b.NM i.d.OPf. (DE)
(74) Vertreter: Hauck Patent- und Rechtsanwälte PartmbB

(56) Entgegenhaltungen:
- DE-A1- 102012 211 816
- DE-U1- 202008 014 892
- GB-A- 2 505 524
- US-B2- 8 537 373

## Beschreibung

Die Erfindung betrifft einen Multilamellenkollimator mit mehreren Lamellen zum Anpassen eines Behandlungsstrahls eines Bestrahlungsgeräts an die Form eines zu behandelnden Objektes, wobei die Lamellen relativ zu dem Behandlungsstrahl entlang einer Bewegungsachse verstellbar sind.

Derartige Multilamellenkollimatoren, im Englischen als Multi-Leaf-Collimator (MLC) bezeichnet, werden zumeist in der Strahlentherapie als Strahlformer verwendet. Im Rahmen einer solchen Strahlentherapie wird ein auf einem Behandlungstisch eines Bestrahlungsgeräts liegender Patient mittels der Bestrahlungsquelle des Bestrahlungsgeräts bestrahlt. Die Bestrahlungsquelle kann beispielsweise ein Linearbeschleuniger sein. Durch eine solche Bestrahlung können beispielsweise Krebsgeschwüre und Tumore oder auch nur Teile derselben als zu behandelnde Objekte behandelt werden, wobei das erkrankte Gewebe durch die Bestrahlung zerstört wird. Um die Beschädigung des umliegenden, gesunden Gewebes zu vermeiden, ist es wichtig, das zu bestrahlende Objekt möglichst exakt zu treffen. Zu diesem Zweck wird an die Bestrahlungsquelle ein Multilamellenkollimator angebracht. Dieser passt den Behandlungsstrahl möglichst genau an die Form des zu behandelnden Objektes an und schattet das umgebende Gewebe ab. Das somit gebildete Muster des Behandlungsstrahls kann bspw. durch eine Bedienperson oder eine Steuereinheit vorgegeben werden. Hierzu werden die Lamellen derart in dem Behandlungsstrahl angeordnet, dass die die Lamellen freigebende Öffnung die Form des zu behandelnden Objektes nachbildet.

Um den Behandlungsstrahl möglichst exakt an die Form des zu behandelnden Objektes anzupassen, muss die Position der einzelnen Lamellen genau ermittelbar sein. Eine solche Positionsermittlung der Lamellen erfolgt bisher üblicherweise durch Erfassen der Motorpositionen, der die Lamellen verstellenden Motoren. Hierzu werden beispielsweise Encoder oder Potentiometer verwendet. Dies ist beispielsweise bekannt aus US 2018/035 969 A1 Eine solche indirekte Positionserfassung der Lamellen kann relativ ungenau und fehleranfällig sein. So kann es beispielsweise aufgrund von Getriebeeinflüssen (sogenannter Backlash) oder auch aufgrund der Gewichtskraft der Lamellen zu Abweichungen kommen. Diese Abweichungen werden mit der Zeit aufgrund von Materialermüdung immer größer. Daher ist es bisher häufig üblich ein weiteres, hiervon unabhängiges Messsystem als Redundanz zu verwenden. Teilweise ist dies sogar vorgeschrieben. Dies ist jedoch aufwendig.

Eine weitere Möglichkeit zur Positionserfassung der Lamellen sind Kamerasysteme, welche die durch die Lamellen freigegebene Öffnung erfassen. Auch sind Kamerasysteme bekannt, die an der Vorderkante der Lamellen angeordnete optische Marker erfassen. Eine solche Positionsermittlung ist beispielsweise in US 2007/176 126 A1 beschrieben, wobei über ein Kamerasystem die Bewegung eines auf einer Lamelle angebrachten optischen Markers verfolgt und hieraus eine Verschiebung der Lamellen ermittelt wird. Eine weitere Vorrichtung zur Ermittlung der Lamellenposition mittels eines Kamerasystems unter Verwendung fluoreszierender Markierungen ist aus EP 2 085 117 A1 bekannt. Derartige Kamerasysteme sind jedoch recht aufwendig. Zudem führt eine Anordnung von Messsystemen, wie insbesondere solcher Kamerasysteme, in der Nähe des Bestrahlungsgeräts aufgrund der hochenergetischen Strahlung zu einer Beeinträchtigung des Messergebnisses und einer Verringerung der Lebensdauer der Messsysteme, insbesondere aufgrund von Streustrahlung. Auch können im Rahmen der Behandlung unter Umständen starke Magnetfelder auftreten, die das Messergebnis beeinträchtigen können.

Aus US 8 537 373 B2 und GB 2 505 524 A sind indirekte optische Messverfahren zum Bestimmen der Lamellenposition eines Multilamellenkollimators bekannt. In US 8 537 373 B2 wird vorgeschlagen, an den Lamellen Retroreflektoren und an von den Lamellen getrennten Orten Referenzreflektoren anzubringen. Laserstrahlung einer Laserquelle wird mittels beweglicher Spiegel in einem Scanvorgang über die Lamellen und die Referenzreflektoren geführt. Mittels eines interferometrischen Messaufbaus wird der Zeitunterscheid zwischen an den Retroreflektoren einerseits und den Referenzreflektoren andererseits reflektierter Laserstrahlung ausgewertet. Hieraus wird die relative Position der Lamellen in Bezug auf die fest angeordneten Referenzreflektoren bestimmt. In GB 2 505 524 A wird ebenfalls vorgeschlagen, Laserstrahlung mittels einer Scanvorrichtung in einem Scanvorgang über die Lamellen eines Multilamellenkollimators zu führen. Auf den Lamellen sind jeweils ein Paar Retroreflektoren angeordnet. Außerdem sind neben den Lamellen Referenzreflektoren angeordnet. In GB 2 505 524 A wird vorgeschlagen, die Position der Retroreflektoren und damit die Position der Lamellen anhand von an den Retroreflektoren und den Referenzreflektoren reflektierter Laserstrahlung in einem Triangulationsverfahren zu ermitteln.

Nachteilig an den erläuterten Messverfahren ist, dass diese abhängig von vorhergehenden Kalibrierverfahren bzw. eingespeicherten Referenzwerten sind. So muss die Position der Referenzreflektoren bekannt sein. Unerkannte Veränderungen der Referenzreflektoren beeinflussen das Messergebnis. Das für die Messung erforderliche Scannen der Lamellenoberflächen erfordert außerdem eine großflächige Zugänglichkeit der Lamellen.

Der Erfindung liegt daher die Aufgabe zugrunde, einen Multilamellenkollimator bereitzustellen, der in einfacher und zuverlässiger Weise eine möglichst genaue Bestimmung der Position der Lamellen ermöglicht. Zudem wird ein Verfahren zur Bestimmung der Position der Lamellen eines Multilamellenkollimators vorgeschlagen.

Die Erfindung löst die Aufgabe durch einen Multilamellenkollimator gemäß Anspruch 1 sowie durch ein Verfahren gemäß Anspruch 15. Vorteilhafte Ausgestaltungen sind Gegenstand der abhängigen Ansprüche, der Beschreibung sowie der Figuren.

Der erfindungsgemäße Multilamellenkollimator der eingangs genannten Art umfasst eine optische Messeinrichtung zum Bestimmen der Position der Lamellen auf der Bewegungsachse mittels einer Laufzeitmessung elektromagnetischer Strahlungssignale.

Das erfindungsgemäße Verfahren sieht entsprechend vor, die Position zumindest einer der Lamellen auf einer Bewegungsachse der Lamelle durch eine Laufzeitmessung zu bestimmen.

Die Lamellen des Kollimators werden, wie eingangs erläutert, zum Anpassen des Behandlungsstrahls in dessen Strahlgang hinein- oder aus diesem hinausverfahren. Insbesondere kann eine Vielzahl von Lamellenpaaren vorgesehen sein, wobei die Lamellen eines Lamellenpaares zum Anpassen des Behandlungsstrahls aufeinander zu und voneinander fort bewegbar sind. Auch kann der Kollimator beispielsweise genau ein Lamellenpaar umfassen, wobei die Lamellen insbesondere als Blöcke (auch als "Jaws" bezeichnet) geformt sein können. Durch entsprechende Abschattung kann somit der Behandlungsstrahl beliebig geformt werden. Die Lamellen sind dabei entlang zumindest einer Bewegungsachse verstellbar. Die Lamellen können jedoch auch entlang mehrerer Bewegungsachsen, insbesondere entlang einer Kurvenbahn, verstellbar sein. Entlang der Bewegungsachse werden die Lamellen relativ zu dem Behandlungsstrahl verstellt, also insbesondere weiter in den Behandlungsstrahl hinein verschoben oder aus dem Behandlungsstrahl heraus bewegt. Dabei werden die Lamellen insbesondere quer zu einer Hauptstrahlachse des Behandlungsstrahls in den Behandlungsstrahl verfahren. Die Bewegungsachse oder die Bewegungsachsen können folglich in einem Winkel zu der Hauptstrahlachse stehen.

Erfindungsgemäß wird die Position der Lamellen des Kollimators auf dieser Bewegungsachse über eine Laufzeitmessung bestimmt. Die optische Messeinrichtung ist daher dazu ausgebildet, elektromagnetische Strahlungssignale auszusenden und zu empfangen. Auf Grundlage der Laufzeit dieser Strahlungssignale kann die Position der Lamellen bestimmt werden. Beispielsweise kann die optische Messeinrichtung eine Sende- und Empfangseinheit aufweisen, welche elektromagnetische Strahlung auf eine Rückseite der Lamelle richtet und die von der Rückseite der Lamelle reflektierte Strahlung aufnimmt. Bei bekannter Geschwindigkeit des Strahlungssignals kann aus der Laufzeit der Abstand zwischen der Sende- und Empfangseinheit und der Lamelle berechnet werden. Hieraus kann die exakte Position der Lamelle bestimmt werden. Insbesondere kann die Position aller Lamellen des Kollimators in der erfindungsgemäßen Weise mittels Laufzeitmessung ermittelbar sein. Die optische Messeinrichtung kann ein oder mehrere Sensorelemente umfassen, die beispielsweise durch solche Sende- und Empfangseinheiten realisiert sein können, wie später noch erläutert wird. Die elektromagnetischen Strahlungssignale können beispielsweise Laserpulse sein. Auch kann die Position durch eine Fouriertransformation im Frequenzraum ermittelt werden. Auch dies wird als Laufzeitmessung verstanden. Grundsätzlich meint die Laufzeitmessung vorliegend, dass das Messergebnis durch die Laufzeit der elektromagnetischen Strahlungssignale bestimmt ist. Durch die erfindungsgemäße absolute Messung der Position der Lamelle mittels Laufzeitmessung des Strahlungssignals ist die Verwendung von außerhalb der Lamelle angeordneten Referenzreflektoren nicht erforderlich. In die Positionsbestimmung gehen als Messgrößen erfindungsgemäß vielmehr nur die Laufzeit der von der Sendeeinheit auf die Lamelle ausgesandten und von der Lamelle reflektierten Strahlung sowie die Strahlungsgeschwindigkeit, also die Lichtgeschwindigkeit, ein. Es erfolgt somit im Gegensatz zu dem eingangs erläuterten Stand der Technik keine indirekte Messung der Lamellenposition. Ein Vergleich mit Referenzpositionen bzw. eine Verwendung von Referenzreflektoren kann entfallen. Erfindungsgemäß wird durch die absolute Bestimmung der Lamellenposition ohne Vergleich mit Referenzpositionen eine zusätzliche Information und Redundanz erreicht, insbesondere bei einem Start des Systems in unbekannter Position. Die erfindungsgemäße Positionsbestimmung ist unabhängig von vorhergehenden Kalibrierverfahren bzw. eingespeicherten Referenzwerten. Unerkannte Veränderungen von Komponenten des Systems haben keinen Einfluss auf die Positionsbestimmung. Weiterhin wird ein Schutz vor unerwünschten mechanischen Kollisionen gewährleistet, da die Betriebsgrenzen jederzeit sicher erkannt werden können.

Die Erfindung erlaubt es weiterhin, mit nur einer Projektionsfläche für die Messstrahlung zu arbeiten. Insbesondere ist das Aussenden der Strahlungssignale auf nur einen Messpunkt auf der Lamelle für die erfindungsgemäße direkte und absolute Positionsbestimmung per Laufzeitmessung ausreichend. Ein Scannen der Lamellenoberfläche, wie im Stand der Technik vorgesehen, kann entfallen. Dadurch erfordert die erfindungsgemäße Messanordnung erheblich weniger Raum bzw. eine erheblich geringere Zugänglichkeit der Lamellen für die Strahlungssignale. Es wird eine besonders kompakte Bauweise ermöglicht. Auch erlaubt die Erfindung eine größere Entfernung der Sende- und Empfangseinheiten zum Strahlenfeld eines Bestrahlungsgeräts. Dies führt zu einer geringeren Streustrahlenbelastung und damit einer niedrigeren Ausfallwahrscheinlichkeit bzw. einer größeren Auswahl an gegebenenfalls strahlenempfindlichen Systemen.

Die erfindungsgemäße Laufzeitmessung erlaubt ein direktes Erfassen der Lamellenpositionen in einer exakten Weise. Es kann somit ein hochgenaues Messsystem gebildet werden, da die Strahlungsfrequenz der Strahlungssignale und damit die Laufzeit hochgenau messbar sind. Aufgrund der somit erreichten hohen Genauigkeit und der absoluten Ermittlung der Lamellenposition ist es nicht unbedingt notwendig, ein weiteres Messsystem als Redundanz vorzusehen. Dies vereinfacht den Aufbau wesentlich. Zumindest kann das erfindungsgemäße Messsystem unabhängig von einem zweiten, aufgrund von Normvorgaben eventuell vorgesehenen zweiten Messsystem arbeiten. Das erfindungsgemäße Messsystem ist zudem besonders einfach, da eine direkte Messung der tatsächlichen Position beziehungsweise Bewegung der Lamellen erfolgt und somit im Gegensatz zu den eingangs erläuterten Messsystemen nicht erst in fehleranfälliger Weise auf die tatsächliche Position umgerechnet werden muss. Zudem weist die erfindungsgemäße optische Messeinrichtung im Wesentlichen keine Mechanik und damit keinen Verschleiß oder Montagetoleranzen auf. Je nach Anordnung der Sensorelemente der optischen Messeinrichtung sind insbesondere keine baulichen Änderungen an den Lamellen erforderlich. Aufgrund der verringerten Komplexität im Vergleich zu bekannten Messsystemen ist das erfindungsgemäße Messsystem kosteneffizienter und weniger störungsanfällig.

Nach einer Ausgestaltung laufen die Strahlungssignale über einen Lichtleiter. Grundsätzlich können die Strahlungssignale unmittelbar, also insbesondere durch die Luft, übertragen werden. Nach dieser Ausgestaltung werden die Strahlungssignale jedoch für eine, mehrere oder insbesondere alle der Lamellen über einen Lichtleiter übertragen. Ein solcher Lichtleiter erlaubt einen besonders störungsfreien Strahlgang und damit eine besonders genaue Positionsbestimmung der Lamelle. Zudem ermöglichen die Lichtleiter eine flexible Anordnung der optischen Messeinrichtung beziehungsweise eines oder mehrerer Sensorelemente der optischen Messeinrichtung, da über den Lichtleiter die elektromagnetischen Strahlungssignale beliebig geführt werden können. So können die optische Messeinrichtung beziehungsweise deren Sensorelemente auch in größerer Entfernung von den Lamellen angeordnet und so vor störenden Einflüssen besser geschützt werden.

Nach einer weiteren Ausgestaltung kann die optische Messeinrichtung dazu ausgebildet sein, die elektromagnetischen Strahlungssignale für die Laufzeitmessung auf eine vom Behandlungsstrahl fortweisende Rückseite bzw. Stirnfläche der Lamelle oder auf eine vom Behandlungsstrahl fortweisende Rückseite bzw. Stirnfläche eines sich mit der Lamelle bewegenden Elements zu richten. Durch die erfindungsgemäße Laufzeitmessung und die Möglichkeit, auf ein Scannen der Lamellenoberfläche zu verzichten, und stattdessen Strahlungssignale nur auf einen Messpunkt auf der jeweiligen Lamelle zu richten, können die Strahlungssignale auf die vergleichsweise kleine rückseitige Stirnfläche der Lamelle bzw. des bewegten Elements gerichtet werden. Dadurch können die Strahlungssignale optimal von dem Strahlungsfeld eines Bestrahlungsgeräts getrennt werden. Gleichzeitig ist das Aussenden auf die Lamellenrückseite bzw. Elementrückseite aufgrund des erfindungsgemäß geringen Raumbedarfs erst möglich. Denn an der Rückseite steht aufgrund der üblicherweise dort befindlichen Spindel-Antriebsmechanik zum Verstellen der Lamellenposition nur ein sehr eingeschränkter Raum zur Verfügung.

Nach einer Ausgestaltung weist die optische Messeinrichtung eine Steuereinheit und ein Sensorelement oder mehrere Sensorelemente zum Aussenden und Empfangen der elektromagnetischen Strahlungssignale auf. Die Steuereinheit ist dabei dazu ausgebildet, aus den Laufzeiten der Strahlungssignale die Position der Lamellen zu bestimmen. Die optische Messeinrichtung kann beispielsweise für jede der Lamellen ein eigenes Sensorelement aufweisen. Auch kann für einige oder für alle der Lamellen ein gemeinsames Sensorelement vorgesehen sein, wie später noch erläutert wird. Die Sensorelemente können jeweils einen Sender zum Aussenden der Strahlungssignale und einen Empfänger zum Empfangen der Strahlungssignale aufweisen. Sender und Empfänger können eine Sende- und Empfangseinheit bilden. Auch können Sender und Empfänger jedoch räumlich voneinander getrennt ausgebildet sein.

Nach einer weiteren Ausgestaltung können die Sensorelemente und gegebenenfalls vorgesehene Lichtleiter in einer Ebene angeordnet sein, vorzugsweise in der durch die Lamellen gebildeten Ebene bzw. der Ebene, in der die Lamellen angeordnet sind, also der sogenannten Kollimatorebene. Der Raumbedarf in der Strahlungsrichtung eines Bestrahlungsgeräts, also quer zur Kollimatorebene, ist in der Regel besonders knapp bemessen bzw. soll möglichst wenig durch die Messeinrichtung beansprucht werden, da dieser Raum direkt den für einen Patienten zur Verfügung stehenden Raum beeinflusst. Dies wird durch die genannte Ausgestaltung ermöglicht, die insbesondere durch die erfindungsgemäße Laufzeitmessung möglich ist. Die Systeme nach dem Stand der Technik benötigen dagegen Raum für Komponenten außerhalb der Kollimatorebene.

So kann nach einer diesbezüglichen Ausgestaltung das Sensorelement abseits der Lamellen angeordnet und dazu ausgebildet sein, die Strahlungssignale auf eine Lamelle oder auf ein sich mit der Lamelle bewegendes Element zu richten. Auch kann nach einer alternativen Ausgestaltung das Sensorelement an einer Lamelle oder an einem sich mit der Lamelle bewegenden Element angeordnet und dazu ausgebildet sein, die Strahlungssignale auf ein Referenzobjekt zu richten. Das Sensorelement kann also abseits der Lamelle angeordnet sein, beispielsweise an einem Gehäuse des Kollimators befestigt sein. Das Sensorelement kann hierbei insbesondere eine Sende- und Empfangseinheit aufweisen. Von dem Sender des Sensorelements wird dann das elektromagnetische Strahlungssignal in Richtung der Lamelle ausgesendet, von der Lamelle reflektiert und durch den Empfänger des Sensorelements wieder aufgenommen. Auch kann das Strahlungssignal nicht direkt auf die Lamelle, sondern auf ein sich mit der Lamelle bewegendes Element gerichtet und diesbezüglich eine Laufzeitmessung vorgenommen werden. Auch so kann auf die Bewegung beziehungsweise die Position der Lamelle geschlossen werden. Das sich mit der Lamelle bewegende Element kann beispielsweise ein Antriebselement sein, wie insbesondere eine Spindel oder ein Teil des Getriebes, wie eine Kupplung. Alternativ kann das Sensorelement, welches dann insbesondere ebenfalls als Sende- und Empfangseinheit ausgebildet ist, an der Lamelle oder an dem sich mit der Lamelle bewegenden Element angeordnet sein, wobei dann der Sender des Sensorelements die Strahlungssignale auf ein Referenzobjekt richtet. Die Strahlungssignale werden dann von dem Referenzobjekt reflektiert und durch den Empfänger an der Lamelle aufgenommen. Das Referenzobjekt kann beispielsweise ein Gehäuse des Kollimators sein. Das Sensorelement ist bei dieser Ausgestaltung mit der Lamelle beweglich. Das sich mit der Lamelle bewegende Element kann, wie erwähnt, ein Antriebselement sein. In der ersten Ausgestaltung wird durch eine Bewegung der Lamelle der Abstand zwischen dem Sensorelement und der Lamelle für die Laufzeitmessung berücksichtigt, während bei der zweiten Ausgestaltung der Abstand zwischen dem sich mit der Lamelle bewegende Sensorelement und dem Referenzobjekt für die Laufzeitmessung berücksichtigt wird.

Nach einer diesbezüglichen Ausgestaltung ist das Sensorelement dazu ausgebildet, die Strahlungssignale auf eine vom Behandlungsstrahl fortweisende Rückseite der Lamelle oder auf eine vom Behandlungsstrahl fortweisende Rückseite des sich mit der Lamelle bewegenden Elements zu richten. Das Sensorelement ist hierbei insbesondere als Sende- und Empfangseinheit ausgebildet und abseits des Behandlungsstrahls angeordnet. Diese Ausgestaltung ermöglicht die Laufzeitmessung fernab des Strahlengangs des Behandlungsstrahls und minimiert somit dessen Einflüsse auf das Messergebnis. Die Positionsbestimmung kann somit besonders genau erfolgen.

Nach einer weiteren Ausgestaltung weist das Sensorelement räumlich voneinander getrennte Sender und Empfänger auf, wobei der Sender an der Lamelle oder an einem sich mit der Lamelle bewegenden Element und der Empfänger abseits der Lamelle angeordnet ist oder wobei der Empfänger an der Lamelle oder an einem sich mit der Lamelle bewegenden Element und der Sender abseits der Lamelle angeordnet ist. Das sich mit der Lamelle bewegende Element kann, wie bereits erwähnt, beispielsweise ein Antriebselement sein. Nach dieser Ausgestaltung bewegt sich der Sender oder der Empfänger mit der Lamelle. Es wird über die Laufzeitmessung der Abstand zwischen Sender und Empfänger des Sensorelements bestimmt.

Sind mehrere Sensorelemente vorgesehen, können insbesondere alle Sensorelemente nach einer der erläuterten Ausgestaltungen angeordnet oder ausgebildet sein. Grundsätzlich können die Sensorelemente jedoch auch unterschiedlichen Ausgestaltungen folgen.

Nach einer Ausgestaltung ist das Sensorelement, das sich mit der Lamelle bewegende Element, der Sender oder der Empfänger an einem vom Behandlungsstrahl fortweisenden Ende der Lamelle, insbesondere an einer vom Behandlungsstrahl fortweisenden Rückseite der Lamelle, angeordnet. Nach dieser Ausgestaltung ist das an der Lamelle angeordnete, an der Laufzeitmessung teilnehmende Bauteil in relativ großer Entfernung von dem Behandlungsstrahl und damit der hochenergetischen Strahlungsquelle befindlich. Auch kann das Bauteil grundsätzlich fortweisend vom Behandlungsstrahl anstatt an der Lamelle, an dem sich mit der Lamelle bewegenden Element angeordnet sein. Somit kann die Laufzeitmessung auch bei diesen Ausgestaltungen fernab des Behandlungsstrahls erfolgen und ist somit im Wesentlichen durch diesen ungestört.

Nach einer Ausgestaltung ist anschließend an ein Sensorelement ein Umschalter angeordnet zur Aufteilung des durch das Sensorelement ausgesandten Strahlungssignals in mehrere Teilstrahlen zum Bestimmen der Positionen mehrerer Lamellen. Es kann somit ein Sensorelement zum Bestimmen der Positionen mehrerer Lamellen verwendet werden. Der Umschalter kann beispielsweise ein Multiplexer oder ein Chopper sein zur Erzeugung der Teilstrahlen. Auch kann der Umrichter oder eine zusätzliche Einheit dazu ausgebildet sein, die elektromagnetischen Strahlungssignale nach Frequenzen aufzuspalten oder zu selektieren. Es kann somit ein Frequenzmultiplexverfahren durchgeführt werden. Insbesondere kann lediglich ein Sensorelement für alle Lamellen des Kollimators vorgesehen sein. Grundsätzlich können jedoch auch mehrere solcher Sensorelemente mit Umschalter sowie auch Sensorelemente, die lediglich die Position einer einzigen Lamelle bestimmen, vorgesehen sein. Nach einer Ausgestaltung werden die Teilstrahlen über Lichtleiter zu den jeweiligen Lamellen geführt. Die Lichtleiter erlauben in besonders einfacher Weise eine störungsfreie Führung der Teilstrahlen von dem Sensorelement zu den jeweiligen Lamellen.

Wie bereits erwähnt, kann der Multilamellenkollimator nach einer Ausgestaltung eine Vielzahl von Lamellenpaaren umfassen, wobei die Lamellen eines Lamellenpaares zum Anpassen des Behandlungsstrahls aufeinander zu und voneinander fort bewegbar sind. Je mehr Lamellenpaare der Kollimator aufweist, desto exakter kann die Anpassung der Form des Behandlungsstrahls erfolgen. Beispielsweise kann der Multilamellenkollimator vier oder mehr Lamellenpaare aufweisen. Alternativ kann der Multilamellenkollimator genau ein Lamellenpaar umfassen, wobei die Lamellen des Lamellenpaares zum Anpassen des Behandlungsstrahls aufeinander zu und voneinander fort bewegbar sind. Ein solcher Kollimator kann insbesondere dazu dienen, den Behandlungsstrahl über seine gesamte Ausdehnung entlang einer Achse beidseitig abzuschatten. Die Lamellen des einen Paares werden dann auch als Blöcke oder "Jaws" bezeichnet. Insbesondere kann auch ein System vorgesehen sein mit zwei Multilamellenkollimatoren, wobei ein zweiter Multilamellenkollimator eine Vielzahl von Lamellenpaaren, insbesondere mindestens vier Lamellenpaare, und ein erster Multilamellenkollimator genau ein Lamellenpaar umfasst. Dabei kann der erste Multilamellenkollimator im Strahlgang des Behandlungsstrahls insbesondere vor dem zweiten Multilamellenkollimator angeordnet sein und den Behandlungsstrahl vorformen während der zweite Multilamellenkollimator den vorgeformten Behandlungsstrahl anschließend detaillierter formt.

Die Erfindung betrifft zudem ein Bestrahlungsgerät umfassend eine Bestrahlungsquelle, einen Behandlungstisch sowie einen erfindungsgemäßen Multilamellenkollimator.

Das erfindungsgemäße Verfahren kann mit dem erfindungsgemäßen Multilamellenkollimator ausgeführt werden. Der Multilamellenkollimator ist also ausgebildet, das erfindungsgemäße Verfahren auszuführen. Die zu dem Multilamellenkollimator gemachten Ausführungen gelten entsprechend für das Verfahren.

Ausgestaltungen der Erfindung werden im Folgenden anhand von Figuren erläutert. Es zeigen:
- Figur 1: ein Bestrahlungsgerät mit einem Multilamellenkollimator
- Figur 2: eine erste Ausgestaltung eines erfindungsgemäßen Multilamellenkollimators in einer Draufsicht, und
- Figur 3: eine zweite Ausgestaltung eines erfindungsgemäßen Multilamellenkollimators in einer Draufsicht.

Soweit nichts anderes angegeben ist, bezeichnen gleiche Bezugszeichen gleiche Gegenstände.

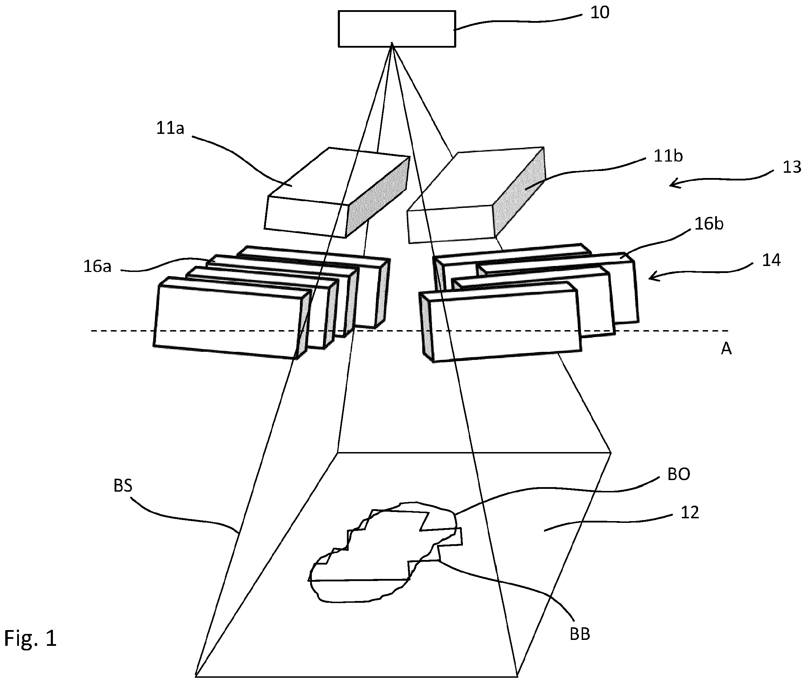

In Figur 1 ist ein Bestrahlungsgerät ersichtlich mit einer Bestrahlungsquelle 10, welche einen Behandlungsstrahl B auf einen Behandlungstisch 12 aussendet. In den Behandlungsstrahl B ragen in Strahlrichtung aufeinanderfolgend zunächst ein Paar im Folgenden als Jaws 11a, 11b bezeichneter Lamellen als Teil eines ersten Multilamellenkollimators 13 und anschließend Lamellen 16 eines zweiten Multilamellenkollimators 14. Jede der in Figur 1 links angeordneten Lamellen 16a bildet mit einer in Figur 1 rechts angeordneten Lamelle 16b ein Lamellenpaar. Die Lamellenpaare 16 können entlang der Bewegungsachse A in den Behandlungsstrahl BS hinein und aus diesem heraus verstellt werden. Hierfür können die Lamellen 16a und 16b eines Lamellenpaares aufeinander zu oder voneinander fortbewegt werden. Ebenso können die Lamellen 11a, 11b des ersten Kollimators 13 in den Behandlungsstrahl B verstellt werden. Die Lamellen schatten den Behandlungsstrahl somit teilweise ab und können diesen an die Form eines zu behandelnden, auf dem Behandlungstisch angeordneten Objektes BO anpassen. Der erste Kollimator 13 dient der groben Vorformung des Behandlungsstrahls während der zweite Kollimator 14 eine exaktere Anpassung an das zu behandelnde Objekt ermöglicht. So ergibt sich auf dem Behandlungstisch der Bestrahlungsbereich BB. Der Bestrahlungsbereich BB deckt dabei nicht das gesamte Objekt BO, sondern nur einen zu behandelnden Teil des Objekts BO ab.

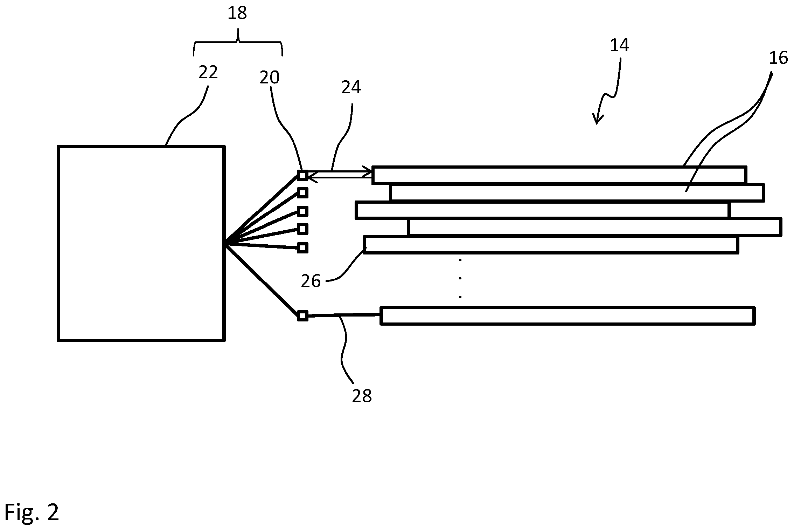

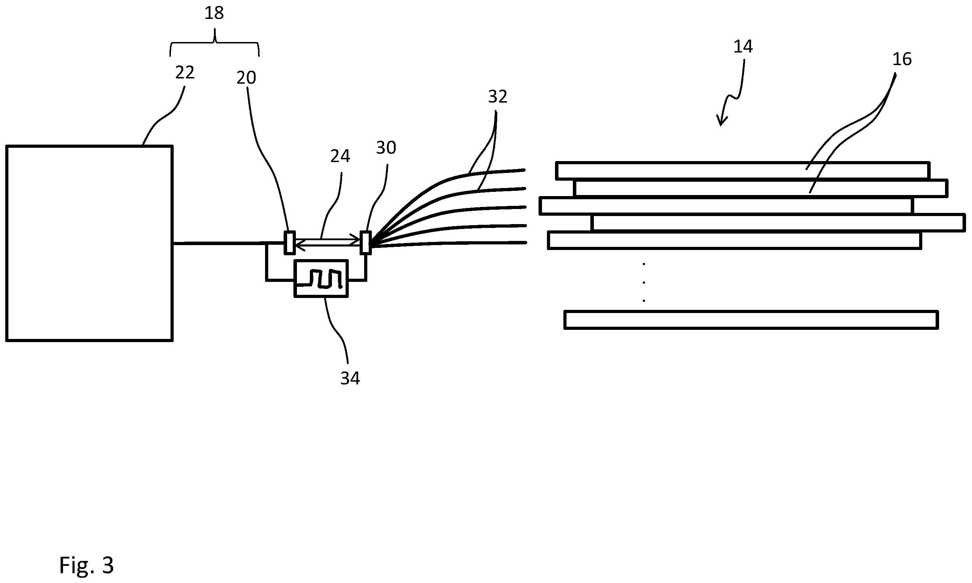

Um den Behandlungsstrahl möglichst genau an die Form des zu behandelnden Objektes bzw. Objektteils anpassen zu können, muss die Position der Lamellen exakt bestimmbar sein. Hierfür ist die erfindungsgemäße, in den Figuren 2 und 3 dargestellte, optische Messeinrichtung 18 vorgesehen. Die Messeinrichtung ist dabei bezogen auf Figur 1 links der linksseitigen Lamellen 16a angeordnet. Die gegenüberliegenden Lamellen des Lamellenpaares können ebenfalls solche Messsysteme aufweisen. Ebenso können auch die Lamellen 11a, 11b des ersten Kollimators 13 eine solche Messeinrichtung aufweisen.

In der Ausgestaltung in Figur 2 umfasst die optische Messeinrichtung 18 mehrere Sensorelemente 20, nämlich für jede der Lamellen 16 ein Sensorelement, sowie eine Steuereinheit 22. In dieser Ausgestaltung sind die Sensorelemente 20 abseits der Lamellen 16 angeordnet und richten elektromagnetische Strahlungssignale 24 jeweils auf eine vom Behandlungsstrahl fortweisende Rückseite 26 der Lamellen 16. Die Lamellen können auf der Rückseite eine reflektierende Beschichtung aufweisen. Die Sensorelemente 20 sind in dieser Ausgestaltung als Sende- und Empfangseinheiten ausgebildet, Sender und Empfänger sind also räumlich in nächster Nähe angeordnet. Die Sensorelemente senden jeweils über ihre Sender ein elektromagnetische Strahlungssignal in Richtung der Rückseite 26 der jeweiligen Lamelle 16, das Strahlungssignal wird an der Rückseite 26 der jeweiligen Lamelle 16 reflektiert und durch den jeweiligen Empfänger der Sensorelemente 20 aufgenommen. Aus der Laufzeit der Strahlungssignale für den Hin- und Rückweg kann bei bekannter Lichtgeschwindigkeit der Abstand zwischen dem jeweiligen Sensorelement und der jeweiligen, dem Sensorelement gegenüberliegenden Lamelle 16 bestimmt werden. Somit kann die Position der Lamelle exakt bestimmt werden.

Die Strahlungssignale können dabei unmittelbar oder über einen lediglich beispielhaft dargestellten Lichtleiter 28 zwischen Sensorelement 20 und Lamelle 16 übertragen werden.

In der Ausgestaltung in Figur 3 ist lediglich ein einziges Sensorelement 20 als Teil der optischen Messeinrichtung 18 vorgesehen. Anschließend an das Sensorelement 20 ist ein Umschalter 30 angeordnet zur Aufteilung des durch das Sensorelement 20 ausgesandten Strahlungssignals in mehrere Teilstrahlen. Die Teilstrahlen werden jeweils über Lichtleiter 32 zu den einzelnen Lamellen 16 geführt. Der Umschalter 30 kann beispielsweise ein Multiplexer oder ein Chopper sein. Bei Bezugszeichen 34 ist eine Vorrichtung zum Synchronisieren dargestellt, die den Umschalter 30 mit dem aus der Steuereinheit kommenden elektrischen Steuersignal zur Unterscheidung der reflektierten Signale und Zuordnung derselben zu den jeweiligen Lamellen synchronisiert.

In beiden Ausgestaltungen ermittelt die Steuereinheit 22 aus den Laufzeiten der Strahlungssignale 24 beziehungsweise der Teilstrahlen die Position der jeweiligen Lamellen 16. Dies geschieht, wie ersichtlich, durch eine direkte Messung und hochgenau, da die Strahlungsfrequenz und damit Laufzeit der Strahlung hochgenau messbar sind. Das gesamte Messsystem kann dadurch, dass die Strahlungssignale auf die Rückseiten der Lamellen gerichtet werden, außerhalb des Behandlungsstrahls angeordnet werden. Durch die Anordnung des Messsystems fernab des Strahlengangs des Behandlungsstrahls werden die Messergebnisse und die Sensorik weniger beeinträchtigt. Fernab bezeichnet hier insbesondere die Anordnung in relativ großer Entfernung von dem Behandlungsstrahl bezogen auf die Bewegungsachse A. Somit ergeben sich eine besonders genaue Positionsbestimmung der Lamellen sowie eine erhöhte Lebensdauer des Messsystems. Aufgrund des einfachen Aufbaus ist das Messsystem grundsätzlich weniger fehleranfällig und von längerer Haltbarkeit, so ist keine Mechanik vorhanden, es gibt wenig Verschleiß und kaum Montagetoleranzen. Ein weiterer Vorteil der erläuterten Ausgestaltungen liegt darin, dass das Lamellendesign unabhängig von dem Messsystem gewählt werden kann, insbesondere ist keine oder kaum eine bauliche Veränderung an den Lamellen erforderlich.

### Bezugszeichenliste

- 10: Bestrahlungsquelle
- 11: Jaws
- 12: Behandlungstisch
- 13: erster Multilamellenkollimator
- 14: zweiter Multilamellenkollimator
- 16, 16a, 16b: Lamellen
- 18: optische Messeinrichtung
- 20: Sensorelemente
- 22: Steuereinheit
- 24: Strahlungssignale
- 26: Rückseite der Lamellen
- 28: Lichtleiter
- 30: Umschalter
- 32: Lichtleiter
- 34: Vorrichtung zum Synchronisieren
- A: Bewegungsachse
- BB: Bestrahlungsbereich
- BO: zu behandelndes Objekt
- BS: Behandlungsstrahl

## Patentansprüche

1. Multilamellenkollimator mit mehreren Lamellen (16) zum Anpassen eines Behandlungsstrahls (B) eines Bestrahlungsgeräts an die Form eines zu behandelnden Objektes, wobei die Lamellen (16) relativ zu dem Behandlungsstrahl (B) entlang einer Bewegungsachse (A) verstellbar sind, umfassend eine optische Messeinrichtung (18) zum Bestimmen der Position der Lamellen (16) auf der Bewegungsachse (A) mittels einer Laufzeitmessung elektromagnetischer Strahlungssignale (24), **dadurch gekennzeichnet, dass** durch die optische Messeinrichtung (18) eine absolute Positionsbestimmung der Lamellen mittels der Laufzeitmessung erfolgt.

2. Multilamellenkollimator nach Anspruch 1, **dadurch gekennzeichnet, dass** die Strahlungssignale (24) über einen Lichtleiter (28, 32) laufen.

3. Multilamellenkollimator nach einem der vorhergehenden Ansprüche, **dadurch gekennzeichnet, dass** die optische Messeinrichtung (18) dazu ausgebildet ist, die elektromagnetischen Strahlungssignale (24) für die Laufzeitmessung auf eine vom Behandlungsstrahl (B) fortweisende Rückseite (26) der Lamelle (16) oder auf eine vom Behandlungsstrahl (B) fortweisende Rückseite (26) eines sich mit der Lamelle (16) bewegenden Elements zu richten.

4. Multilamellenkollimator nach einem der vorhergehenden Ansprüche, **dadurch gekennzeichnet, dass** die optische Messeinrichtung (18) eine Steuereinheit (22) und ein oder mehrere Sensorelemente (20) zum Aussenden und Empfangen der elektromagnetischen Strahlungssignale (24) aufweist, wobei die Steuereinheit (22) aus den Laufzeiten der Strahlungssignale (24) die Position der Lamellen (16) bestimmt.

5. Multilamellenkollimator nach Anspruch 4, **dadurch gekennzeichnet, dass** die Sensorelemente (20) in einer Ebene angeordnet sind, vorzugsweise in der durch die Lamellen (16) gebildeten Ebene.

6. Multilamellenkollimator nach einem der Ansprüche 4 oder 5, **dadurch gekennzeichnet, dass** das Sensorelement (20) abseits der Lamellen (16) angeordnet und dazu ausgebildet ist, die Strahlungssignale (24) auf eine Lamelle (16) oder auf ein sich mit der Lamelle (16) bewegendes Element zu richten.

7. Multilamellenkollimator nach Anspruch 6, **dadurch gekennzeichnet, dass** das Sensorelement (20) dazu ausgebildet ist, die Strahlungssignale (24) auf eine vom Behandlungsstrahl (B) fortweisende Rückseite (26) der Lamelle (16) oder auf eine vom Behandlungsstrahl (B) fortweisende Rückseite (26) des sich mit der Lamelle (16) bewegenden Elements zu richten.

8. Multilamellenkollimator nach einem der Ansprüche 4 oder 5, **dadurch gekennzeichnet, dass** das Sensorelement an der Lamelle oder an einem sich mit der Lamelle bewegenden Element angeordnet und dazu ausgebildet ist, die Strahlungssignale jeweils auf ein Referenzobjekt zu richten.

9. Multilamellenkollimator nach einem der Ansprüche 4 oder 5, **dadurch gekennzeichnet, dass** das Sensorelement räumlich voneinander getrennte Sender und Empfänger aufweist, wobei der Sender an der Lamelle oder an einem sich mit der Lamelle bewegenden Element und der Empfänger abseits der Lamelle angeordnet ist oder wobei der Empfänger an der Lamelle oder an einem sich mit der Lamelle bewegenden Element und der Sender abseits der Lamelle angeordnet ist.

10. Multilamellenkollimator nach einem der Ansprüche 6 bis 9, **dadurch gekennzeichnet, dass** das sich bewegende Element ein Antriebselement ist.

11. Multilamellenkollimator nach einem der Ansprüche 8 bis 10, **dadurch gekennzeichnet, dass** das Sensorelement, das sich mit der Lamelle bewegende Element, der Sender oder der Empfänger an einem vom Behandlungsstrahl fortweisenden Ende der Lamelle angeordnet ist.

12. Multilamellenkollimator nach einem der Ansprüche 4 bis 11, **dadurch gekennzeichnet, dass** anschließend an ein Sensorelement (20) ein Umschalter (30) angeordnet ist zur Aufteilung des durch das Sensorelement (20) ausgesandten Strahlungssignals (24) in mehrere Teilstrahlen zum Bestimmen der Positionen mehrerer Lamellen (16).

13. Multilamellenkollimator nach Anspruch 12, **dadurch gekennzeichnet, dass** die Teilstrahlen über Lichtleiter (32) zu den jeweiligen Lamellen (16) geführt werden.

14. Bestrahlungsgerät umfassend eine Bestrahlungsquelle (10), einen Behandlungstisch (12) sowie einen Multilamellenkollimator (14) nach einem der vorhergehenden Ansprüche.

15. Verfahren zur Bestimmung der Position einer Lamelle (16) eines Multilamellenkollimators (14), wobei die Position zumindest einer der Lamellen (16) auf einer Bewegungsachse (A) der Lamelle (16) durch eine Laufzeitmessung bestimmt wird, **dadurch gekennzeichnet, dass** eine absolute Positionsbestimmung der Lamellen durch eine optische Messeinrichtung (18) mittels der Laufzeitmessung erfolgt

## Claims

1. Multilamellar collimator with several leaves (16) for adapting a treatment beam (B) of an irradiation device to the shape of an object to be treated, wherein the leaves (16) are adjustable relative to the treatment beam (B) along an axis of movement (A), comprising an optical measuring device (18) for determining the position of the leaves (16) on the axis of movement (A) by means of a time-of-flight measurement of electromagnetic radiation signals (24), **characterized in that** an absolute position determination of the leaves is carried out by the optical measuring device (18) by means of the time-of-flight measurement.

2. Multilamellar collimator according to claim 1, **characterized in that** the radiation signals (24) travel via a light guide (28, 32).

3. Multilamellar collimator according to one of the preceding claims, **characterized in that** the optical measuring device (18) is designed to direct the electromagnetic radiation signals (24) for the time-of-flight measurement onto a rear side (26) of the leaf (16) facing away from the treatment beam (B) or onto a rear side (26) of an element moving with the leaf (16) facing away from the treatment beam (B).

4. Multilamellar collimator according to one of the preceding claims, **characterized in that** the optical measuring device (18) has a control unit (22) and one or more sensor elements (20) for transmitting and receiving the electromagnetic radiation signals (24), wherein the control unit (22) determines the position of the leaves (16) from the times of flight of the radiation signals (24).

5. Multilamellar collimator according to claim 4, **characterized in that** the sensor elements (20) are arranged in a plane, preferably in the plane formed by the leaves (16).

6. Multilamellar collimator according to one of claims 4 or 5, **characterized in that** the sensor element (20) is arranged remote from the leaves (16) and is designed to direct the radiation signals (24) onto a leaf (16) or onto an element moving with the leaf (16).

7. Multilamellar collimator according to claim 6, **characterized in that** the sensor element (20) is designed to direct the radiation signals (24) onto a rear side (26) of the leaf (16) facing away from the treatment beam (B) or onto a rear side (26) of the element moving with the leaf (16) facing away from the treatment beam (B).

8. Multilamellar collimator according to one of claims 4 or 5, **characterized in that** the sensor element is arranged on the leaf or on an element moving with the leaf and is designed to direct the radiation signals in each case onto a reference object.

9. Multilamellar collimator according to one of claims 4 or 5, **characterized in that** the sensor element has spatially separated transmitter and receiver, wherein the transmitter is arranged on the leaf or on an element moving with the leaf and the receiver is arranged remote from the leaf, or wherein the receiver is arranged on the leaf or on an element moving with the leaf and the transmitter is arranged remote from the leaf.

10. Multilamellar collimator according to one of claims 6 to 9, **characterized in that** the moving element is a drive element.

11. Multilamellar collimator according to one of claims 8 to 10, **characterized in that** the sensor element, the element moving with the leaf, the transmitter or the receiver is arranged at an end of the leaf facing away from the treatment beam.

12. Multilamellar collimator according to one of claims 4 to 11, **characterized in that** downstream of a sensor element (20), a switch (30) is arranged for splitting the radiation signal (24) transmitted by the sensor element (20) into multiple partial beams for determining the positions of multiple leaves (16).

13. Multilamellar collimator according to claim 12, **characterized in that** the partial beams are guided via light guides (32) to the respective leaves (16).

14. Irradiation device comprising a radiation source (10), a treatment table (12) as well as a multilamellar collimator (14) according to one of the preceding claims.

15. Method for determining the position of a leaf (16) of a multilamellar collimator (14), wherein the position of at least one of the leaves (16) on an axis of movement (A) of the leaf (16) is determined by a time-of-flight measurement, **characterized in that** an absolute position determination of the leaves is carried out by an optical measuring device (18) by means of the time-of-flight measurement.

## Revendications

1. Collimateur à lamelles multiples doté de plusieurs lamelles (16) permettant d'adapter un rayon de traitement (B) d'un appareil d'irradiation à la forme d'un objet à traiter, dans lequel les lamelles (16) sont réglables par rapport au rayon de traitement (B) le long d'un axe de déplacement (A), comportant un dispositif de mesure optique (18) permettant de déterminer la position des lamelles (16) sur l'axe de déplacement (A) au moyen d'une mesure de temps de propagation de signaux de rayonnement électromagnétiques (24), **caractérisé en ce qu'**une détermination de position absolue des lamelles est réalisée par le dispositif de mesure optique (18) au moyen de la mesure de temps de propagation.

2. Collimateur à lamelles multiples selon la revendication 1, **caractérisé en ce que** les signaux de rayonnement (24) passent par un conducteur de lumière (28, 32).

3. Collimateur à lamelles multiples selon l'une des revendications précédentes, **caractérisé en ce que** le dispositif de mesure optique (18) est conçu pour orienter les signaux de rayonnement électromagnétiques (24) pour la mesure de temps de propagation vers un côté arrière (26) de la lamelle (16) opposé au rayon de traitement (B) ou vers un côté arrière (26) opposé au rayon de traitement (B) d'un élément se déplaçant conjointement avec la lamelle (16).

4. Collimateur à lamelles multiples selon l'une des revendications précédentes, **caractérisé en ce que** le dispositif de mesure optique (18) présente une unité de commande (22) et un ou plusieurs éléments de capteur (20) pour l'émission et la réception des signaux de rayonnement électromagnétiques (24), dans lequel l'unité de commande (22) détermine la position des lamelles (16) à partir des temps de propagation des signaux de rayonnement (24).

5. Collimateur à lamelles multiples selon la revendication 4, **caractérisé en ce que** les éléments de capteur (20) sont disposés dans un plan, de préférence dans le plan formé par les lamelles (16).

6. Collimateur à lamelles multiples selon l'une des revendications 4 ou 5, **caractérisé en ce que** l'élément de capteur (20) est disposé à l'écart des lamelles (16) et conçu pour orienter les signaux de rayonnement (24) vers une lamelle (16) ou vers un élément se déplaçant conjointement avec la lamelle (16).

7. Collimateur à lamelles multiples selon la revendication 6, **caractérisé en ce que** l'élément de capteur (20) est conçu pour orienter les signaux de rayonnement (24) vers un côté arrière (26) de la lamelle (16) opposé au rayon de traitement (B) ou vers un côté arrière (26) opposé au rayon de traitement (B) d'un élément se déplaçant conjointement avec la lamelle (16).

8. Collimateur à lamelles multiples selon l'une des revendications 4 ou 5, **caractérisé en ce que** l'élément de capteur est disposé sur la lamelle ou sur un élément se déplaçant conjointement avec la lamelle et conçu pour orienter les signaux de rayonnement respectivement vers un objet de référence.

9. Collimateur à lamelles multiples selon l'une des revendications 4 ou 5, **caractérisé en ce que** l'élément de capteur présente un émetteur et un récepteur spatialement séparés l'un de l'autre, dans lequel l'émetteur est disposé sur la lamelle ou sur un élément se déplaçant conjointement avec la lamelle et le récepteur est disposé à l'écart de la lamelle ou dans lequel le récepteur est disposé sur la lamelle ou sur un élément se déplaçant conjointement avec la lamelle et l'émetteur est disposé à l'écart de la lamelle.

10. Collimateur à lamelles multiples selon l'une des revendications 6 à 9, **caractérisé en ce que** l'élément en déplacement est un élément d'entraînement.

11. Collimateur à lamelles multiples selon l'une des revendications 8 à 10, **caractérisé en ce que** l'élément de capteur, l'élément se déplaçant conjointement avec la lamelle, l'émetteur ou le récepteur est disposé à une extrémité de la lamelle opposée au rayon de traitement.

12. Collimateur à lamelles multiples selon l'une des revendications 4 à 11, **caractérisé en ce qu'**un commutateur (30) est disposé à la suite d'un élément de capteur (20), pour la division du signaux de rayonnement (24) émis par l'élément de capteur (20) en plusieurs rayons partiels pour la détermination des positions de plusieurs lamelles (16).

13. Collimateur à lamelles multiples selon la revendication 12, **caractérisé en ce que** les rayons partiels sont guidés vers les lamelles (16) respectives par le biais de conducteurs de lumière (32).

14. Appareil d'irradiation comportant une source d'irradiation (10), une table de traitement (12) ainsi qu'un collimateur à lamelles multiples (14) selon l'une des revendications précédentes.

15. Procédé de détermination de la position d'une lamelle (16) d'un collimateur à lamelles multiples (14), dans lequel la position de l'une au moins des lamelles (16) sur un axe de déplacement (A) de la lamelle (16) est déterminée par une mesure de temps de propagation, **caractérisé en ce qu'**une détermination de position absolue des lamelles est réalisée par un dispositif de mesure optique (18) au moyen de la mesure de temps de propagation.
